(19) Europäisches Patentamt
European Patent Office
Office européen des brevets

(11) Veröffentlichungsnummer: 0 272 591
A2

(12) # EUROPÄISCHE PATENTANMELDUNG

(21) Anmeldenummer: 87118608.6

(22) Anmeldetag: 15.12.87

(51) Int. Cl.4: **C07D 487/16**, C08K 5/34, //(C07D487/16,251:00,235:00, 235:00)

(30) Priorität: 22.12.86 DE 3643887

(43) Veröffentlichungstag der Anmeldung: 29.06.88 Patentblatt 88/26

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Aumueller, Alexander, Dr.
An der Marlach 5
D-6705 Deidesheim(DE)
Erfinder: Neumann, Peter, Dr.
Franz-Schubert-Strasse 1
D-6908 Wiesloch(DE)
Erfinder: Trauth, Hubert
Milanstrasse 5
D-6724 Dudenhofen(DE)

(54) **Acetylendiharnstoffderivate und ihre Verwendung zum Stabilisieren von Kunststoffen.**

(57) Es werden Acetylendiharnstoffderivate der allgemeinen Formel (I)

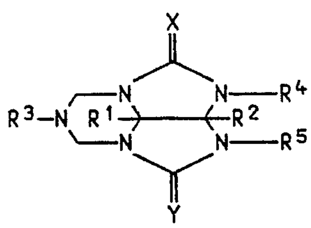

(I),

worin die Substituenten die im Anspruch 1 angegebenen Bedeutungen besitzen, bereitgestellt. Diese Verbindungen eignen sich zum Stabilisieren von organischem Material.

EP 0 272 591 A2

Xerox Copy Centre

## Acetylendiharnstoffderivate und ihre Verwendung zum Stabilisieren von Kunststoffen

Es ist bekannt, daß 2,2,6,6-Tetraalkyl-piperidinderivate Lichtschutzmittel für organische Polymere sind. In der nicht vorveröffentlichten EP-A-213 570 werden Acetylendiharnstoffderivate mit eingebauten 2,2,6,6-Tetraalkylpiperidinresten beschrieben. Es ist ebenfalls bekannt, daß Harnstoff-und Thioharnstoffderivate eine gute Wärmestabilisierung in einigen Kunststoffen erzielen. Jedoch besitzen nur 4-Hydroxyphenyl oder 4-Alkoxyphenylsubstituierte (Thio-)Harnstoffe eine allgemeine UV-Absorberwirkung (vgl. J. Voigt, in K.A. Wolf (Hrsg.), Chemie, Physik und Technologie der Kunststoffe, Springer-Verlag, Berlin, Heidelberg, New York, 1966, S. 300-303).

In der FR-A-2 291 203 sind Glycolurilderivate der Formel

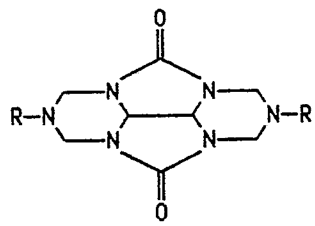

beschrieben, worin R einen gegebenenfalls substituierten Kohlenwasserstoffrest mit bis zu 22 Kohlenstoffatomen bedeutet. Diese Verbindungen werden als oberflächenaktive Mittel für die Textilindustrie und als Korrosionsinhibitoren vorgeschlagen.

Der Erfindung liegt die Aufgabe zugrunde, neue Verbindungen als Stabilisatoren für organisches Material bereitzustellen.

Diese Aufgabe wird gelöst durch die Verbindungen der allgemeinen Formel (I)

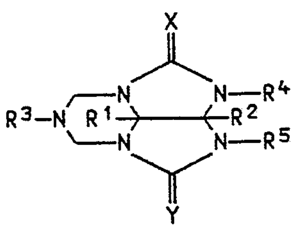

(I),

worin

$R^1$ und $R^2$ unabhängig voneinander Wasserstoff, $C_1$-$C_8$-Alkyl, $C_5$-$C_8$-Cycloalkyl, $C_7$-$C_{12}$-Aralkyl, $C_7$-$C_{14}$-Cycloalkylalkyl, Aryl oder Carbonester bedeuten oder $R^1$ und $R^2$ zusammen eine Tetra-, Penta-oder Hexamethylengruppierung oder einen gegebenenfalls substituierten Rest der Formel

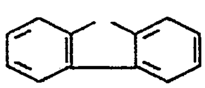

darstellen,

X und Y unabhängig voneinander für Sauerstoff, Schwefel oder $NR^{10}$ stehen, wobei $R^{10}$ Wasserstoff, $C_1$-$C_8$-Alkyl oder $C_7$-$C_{12}$-Aralkyl ist, und

$R^3$ für Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, $C_3$-$C_{12}$-Alkinyl, Hydroxyl, einen nichtaromatischen, gegebenenfalls substituierten Heterocyclus, einen $C_1$-$C_{22}$-Alkylrest, der durch einen Heterocyclus, substituiert ist, einen $C_1$-$C_{22}$-Alkylrest, der durch Amino, Hydroxy, Carboxyl, Carbonester und/oder gegebenenfalls substituiertes Carbamoyl substituiert ist, oder für die Gruppierung B-A-steht, worin -A-ein Brückenglied und B-einen Thiol-oder Cyanrest oder eine Gruppe der Formeln

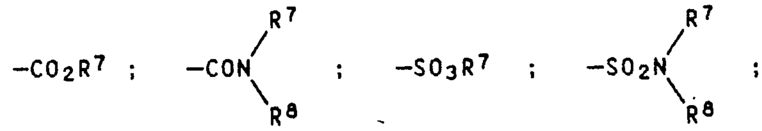

bedeuten, worin

$R^6$ Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl,

$R^7$ und $R^8$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^7$ und $R^8$ gemeinsam mit dem Atom, an das sie gebunden sind, ein 3 bis 12-gliedriges Ringsystem bilden, und

$R^4$ und $R^5$ unabhängig voneinander für Wasserstoff oder $CH_2OR^9$ stehen, worin $R^9$ die Bedeutungen Wasserstoff oder gegebenenfalls substituiertes $C_1$-$C_{22}$-Alkyl, das durch Ethersauerstoff, Stickstoff oder Schwefel unterbrochen sein kann, besitzt,

sowie die Säureadditionssalze und Hydrate dieser Verbindungen.

Gegenstand der Erfindung ist auch die Verwendung der Verbindungen der allgemeinen Formel I zum Stabilisieren von Polyolefinen, Polyamiden und Lacken.

Eine besonders bevorzugte Verwendung ist die zum Licht-und Wärmeschutz und als Metallionendesaktivator, insbesondere bei Kunststoffen.

Einzelne Reste $R^1$ und $R^2$ sind neben Wasserstoff z.B.: Methyl, Ethyl, Propyl, Butyl, Pentyl, Hexyl, Benzyl, Phenylethyl, Phenylpropyl, Phenylbutyl, Methylbenzyl, Phenyl, Tolyl, Carbomethoxy, Carboethoxy, Carbopropoxy oder Carbobutoxy.

Bevorzugt sind für $R^1$ und $R^2$ Methyl, Ethyl, Benzyl, Phenyl, insbesondere Wasserstoff, Methyl und Phenyl. Von diesen ist Wasserstoff besonders bevorzugt.

X und Y sind unabhängig voneinander insbesondere Sauerstoff, daneben auch Schwefel oder $NR^{10}$, worin $R^{10}$ die vorstehenden Bedeutungen besitzt. Alkylreste, beispielsweise $R^3$, können sowohl unverzweigt als auch verzweigt sein.

Alkylreste in den Verbindungen der allgemeinen Formel (I) sind beispielsweise $C_1$-$C_{22}$-Alkylreste, insbesondere $C_1$-$C_8$-Alkylreste, besonders bevorzugt $C_1$-$C_4$-Alkylreste, z.B. Methyl, Ethyl, Propyl, Butyl, Pentyl, Hexyl, Heptyl und Octyl.

Beispiele für solche Reste sind

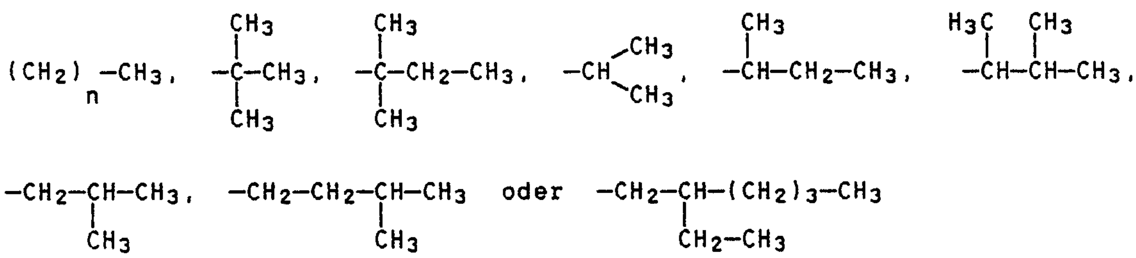

oder

wobei n = 0 - 21 ist.

Ein durch Ethersauerstoff oder Stickstoff unterbrochener Alkylrest z.B. für $R^3$ kann beispielsweise sein:

$CH_3-O-CH_2CH_2-$, $CH_3CH_2-O-CH_2CH_2-$, $CH_3-O-CH_2CH_2CH_2-$, $CH_3CH_2-O-CH_2CH_2CH_2-$,

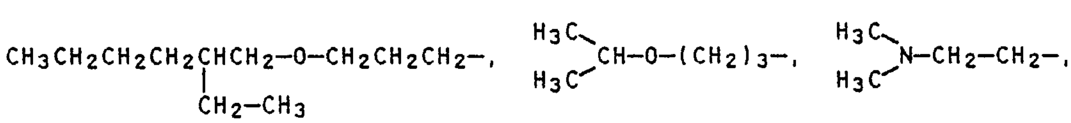

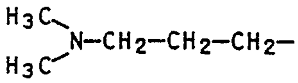, 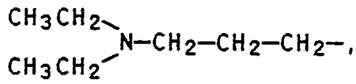, 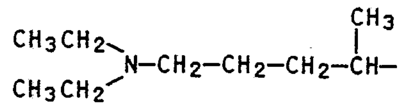,

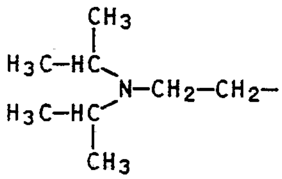 oder 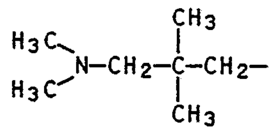 .

Beispielhafte Harnstoffgruppierungen sind die folgenden:

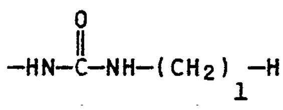, 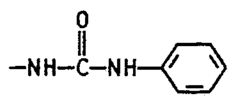, 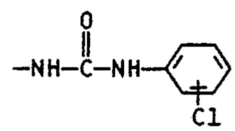,

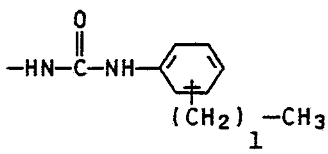, 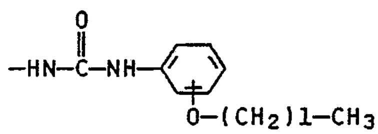,

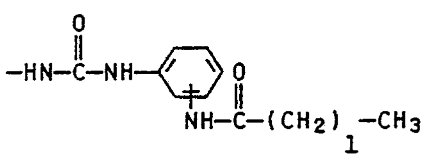, 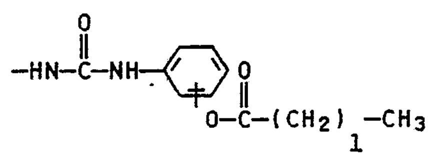,

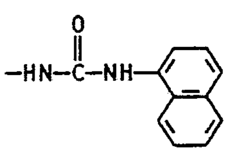, 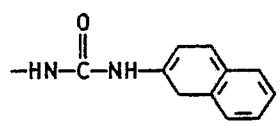,

(l = 0 bis 22).

Beispielhafte Urethangruppierungen bzw. Carbamoylgruppen sind die folgenden:

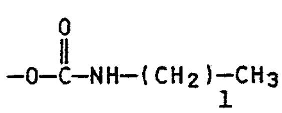, 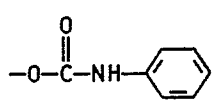, 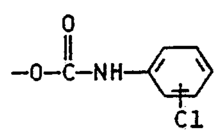, 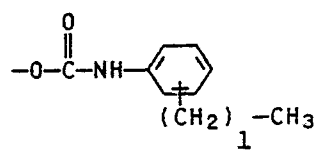

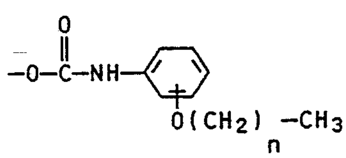, 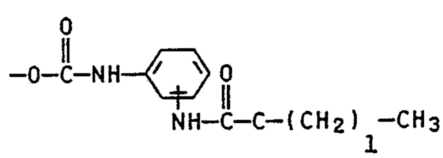,

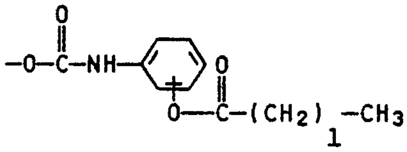 , 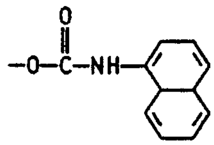 ,

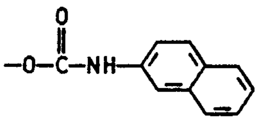 (l = 0 bis 22), 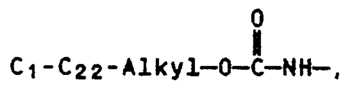

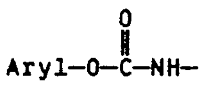 , 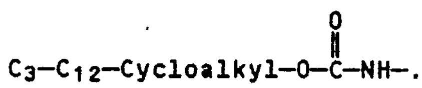

Cycloalkylreste (einschließlich Bicycloalkyl) z.B. für $R^3$ können beispielsweise sein:

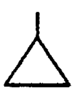 , 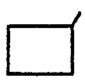 , 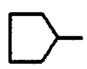 , 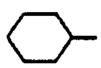 , 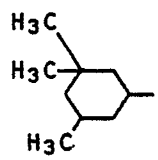 , 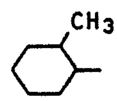 , 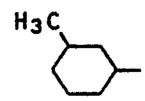 ,

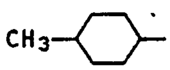 , 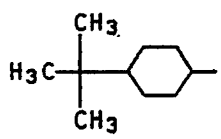 , 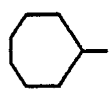 , 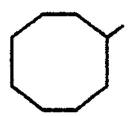 , 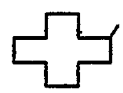 ,

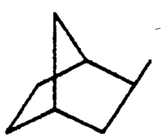 , 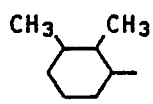 , 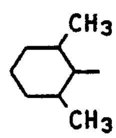 , 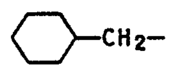 ,

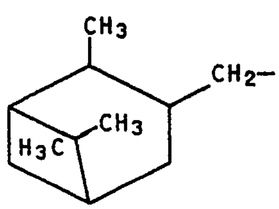 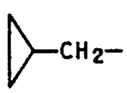 , 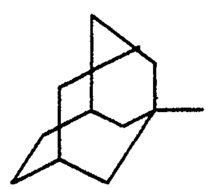

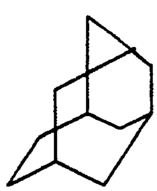 , 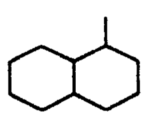 , 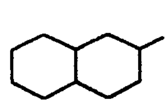 , 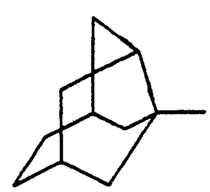

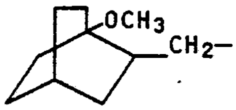 , 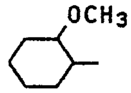 , 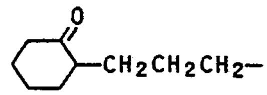 ,

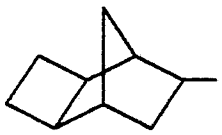 , 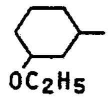 , 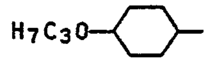 ,

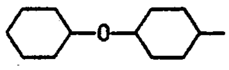 , 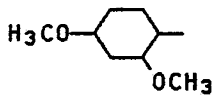 , 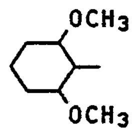 ,

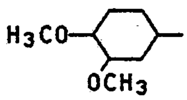 , 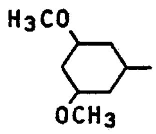 , 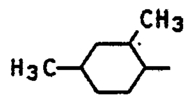 , 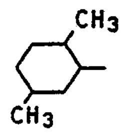

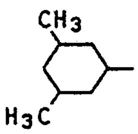 , 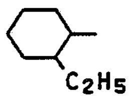 , 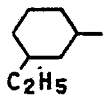 , 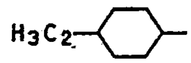 , 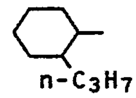 ,

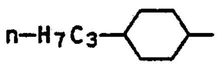 , 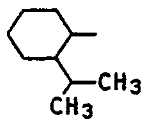 , 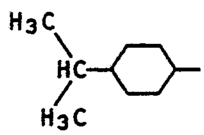 , 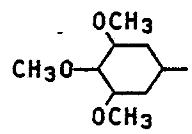 ,

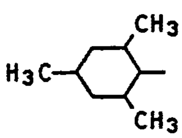 , 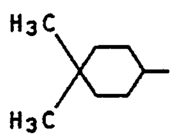 , 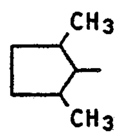 ,

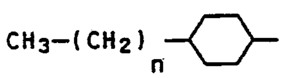 , 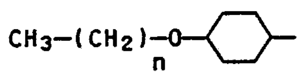 .

(n = 1 bis 20)

Aralkylreste, z.B. $R^3$ können beispielsweise sein:

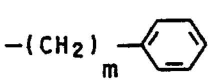 , 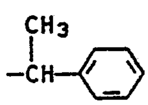 , 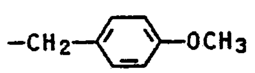 , 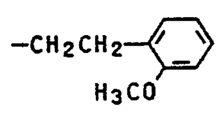 ,

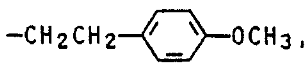, 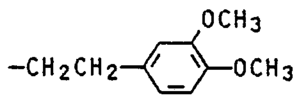, 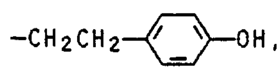, 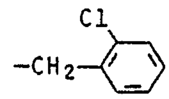,

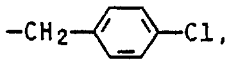, 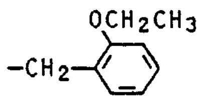 wobei m = 1 bis 6 ist,

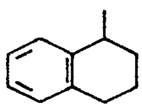, 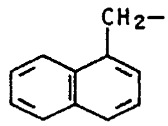, 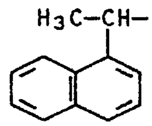, 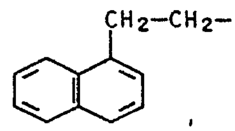,

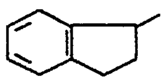, 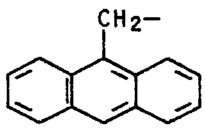, 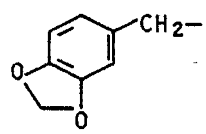, 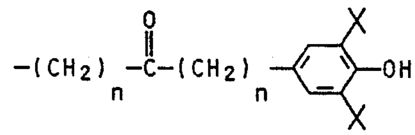,

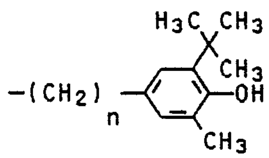, 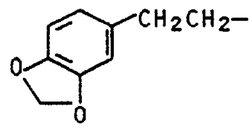, 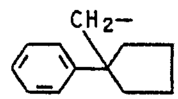,

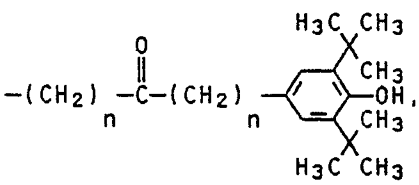, 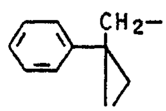, 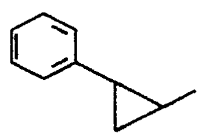, 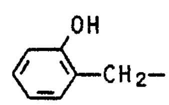,

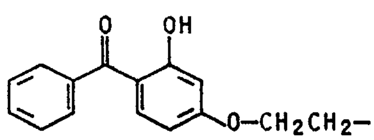, 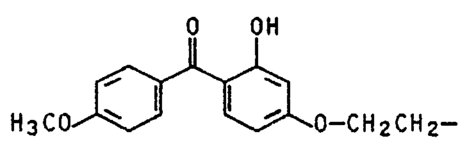,

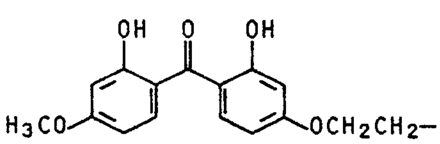, 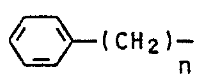 wobei n = 1 bis 20 ist.

Alkylreste, z.B. $R^3$, die Heterocyclen enthalten, können beispielsweise sein:

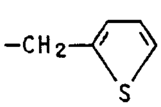, 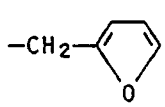, 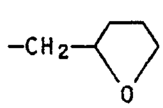, 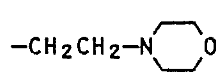, 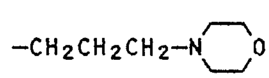,

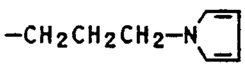 , 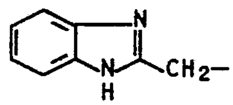 , 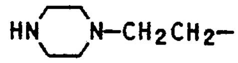 , 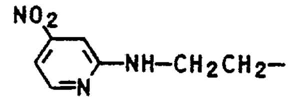 ,

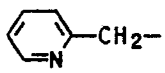 , 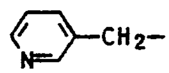 , 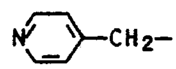 ,

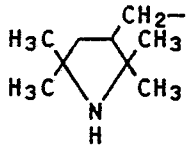 , 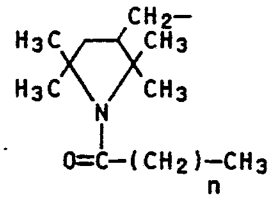 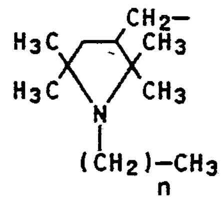 ,

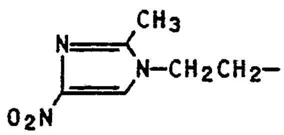 , 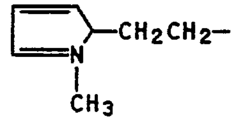 , 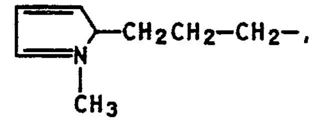,

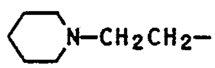 , 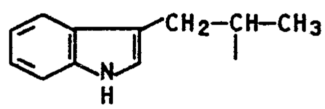 , 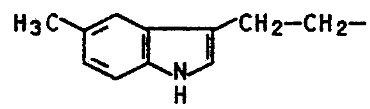 ,

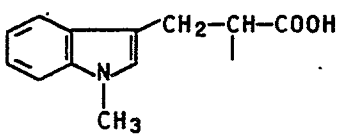 , 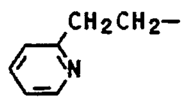 , 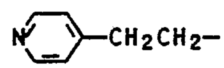 ,

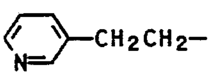 , 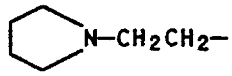 , 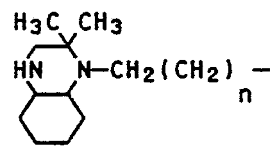 ,

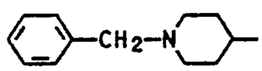 , 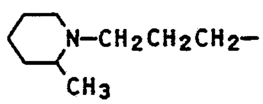 , 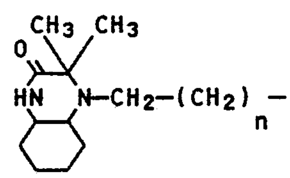 ,

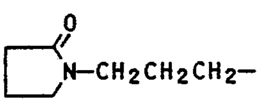 , 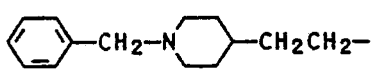 , 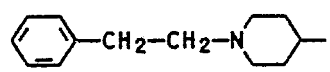 ,

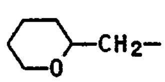 , 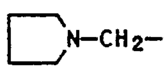 , 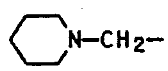 ,

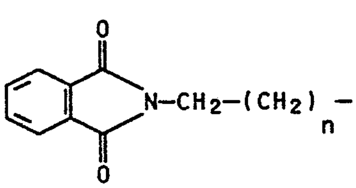, 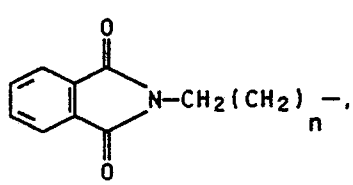, 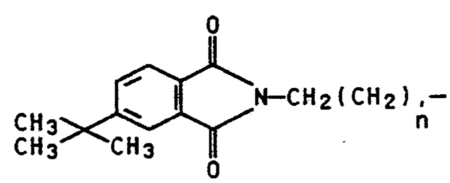

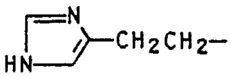 , 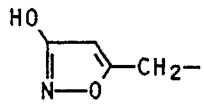 , 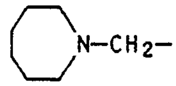 , 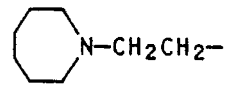 ,

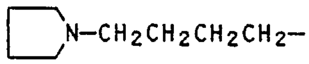 , 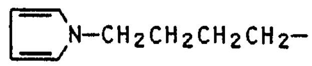 , 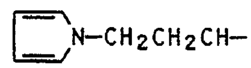 ,

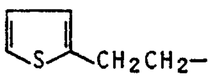 , 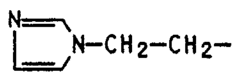 , 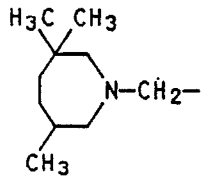 , 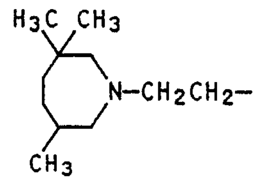 ,

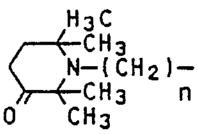 , 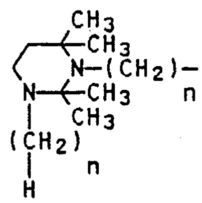 , 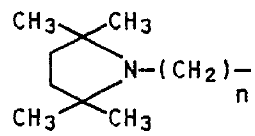 ,

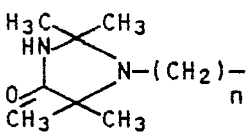 , wobei n = 1 bis 20 ist.

Alkylreste, z.B. $R^3$, die Hydroxyl-, Thiol-, Carboxyl- oder Cyangruppen enthalten, können beispielsweise sein:

$-CH_2CH_2-OH$, $-CH_2CH_2CH_2-OH$, 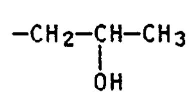, 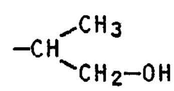, 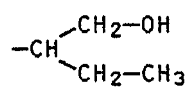,

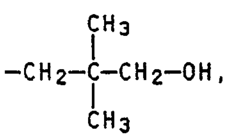, $-CH_2CH_2-SH$, $-(CH_2)_n-COOH$, 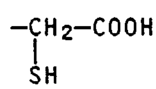, 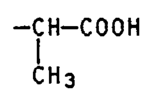, 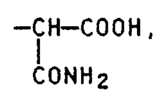,

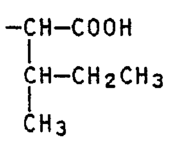 , 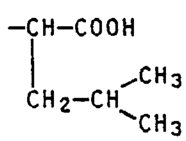 , 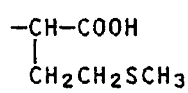 , 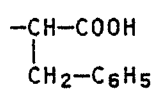 , 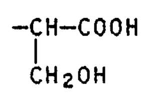 ,

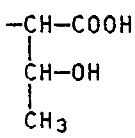 , 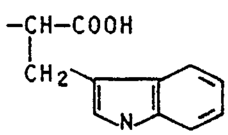 , 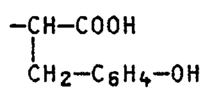 , 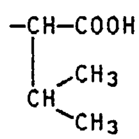 , 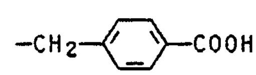 ,

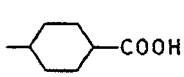 , $-(CH_2)_n-CN$ oder 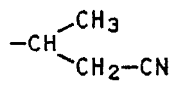 wobei n = 1 bis 20 ist.

Soweit in den erfindungsgemäßen Verbindungen nichtaromatische Heterocyclen vorliegen, können diese beispielsweise die folgenden sein:

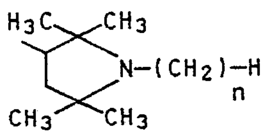 , 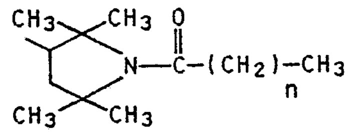 , 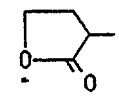 ,

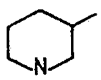 , 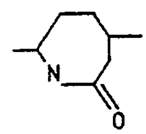 , 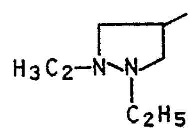 , 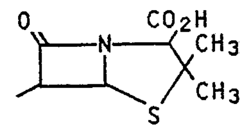 ,

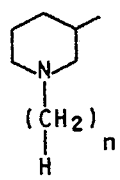 , 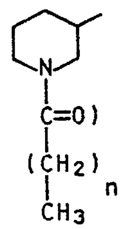 , 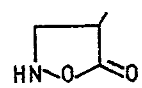 , 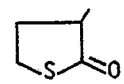 ,

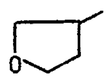 , 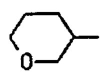 , 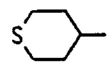 , 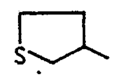 ,

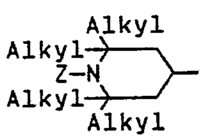 (Z = Wasserstoff, Alkyl, C-Acyl, HO—)

Soweit in den erfindungsgemäßen Verbindungen Hydroxy-substituierte oder Carboxyl-substituierte Alkylreste vorliegen, können diese beispielsweise die folgenden sein:

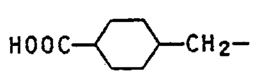 , $HOOC-(CH_2)_n-$ (n = 1 bis 20) , 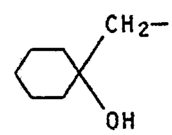 , 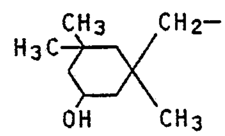 ,

$HO-CH_2CH_2O-CH_2CH_2-$ , $HO-(CH_2)_n-$ (n = 1 bis 20), $H-(CH_2)_n-\overset{O}{\overset{\|}{C}}NH-(CH_2)_m-$ ,

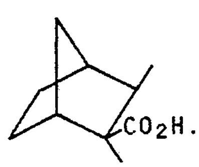

Brückenglieder -A-sind zweiwertige aliphatische, araliphatische oder aromatische Gruppen, die auch

Heteroatome, nämlich Sauerstoff, Stickstoff oder Schwefel, enthalten können.

Insbesondere sind Alkylen-, Cycloalkylen-, Aralkylen-, durch CO oder $SO_2$ substituierte Alkylen-, Aralkylen-oder Arylreste zu nennen.

Einzelne Brückenglieder sind beispielsweise:

$-(CH_2)_p-$, $-(CH_2)_pCH{=}CH-$, $-(CH_2)_p-C{\equiv}C-$, 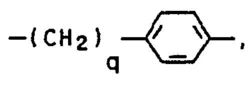, 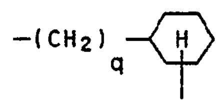,

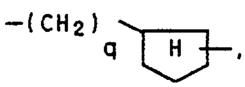, 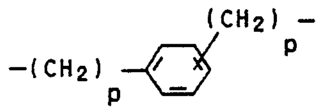, $-(CH_2)_2O-$, $-(CH_2)_2O(CH_2)_2-$,

$-(CH_2)_3O(CH_2)_2-$, 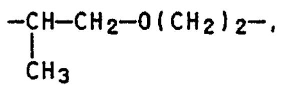, 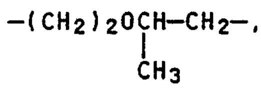, 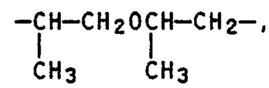

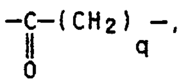, 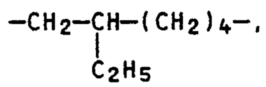, 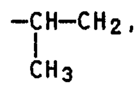, $-CH_2-CH{=}CH-$, 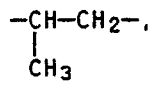, 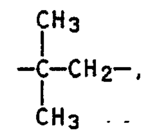

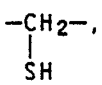, 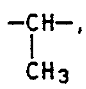, 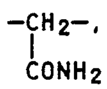, 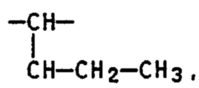, 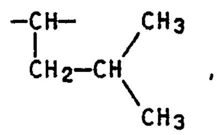, 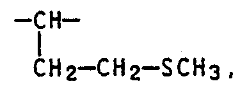

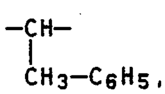, 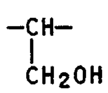, 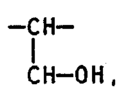, 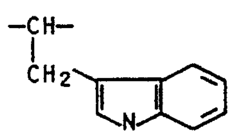 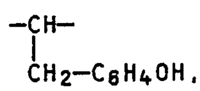, 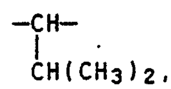

wobei p = 1 bis 20 und q = 0 bis 4.

Bevorzugte Brückenglieder sind $-(CH_2)-_n$, worin n = 1 bis 5, insbesondere 1, 2 und 5 ist.

Weitere Beispiele für Brückenglieder -B- sind die folgenden:

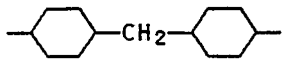 , 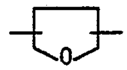 , 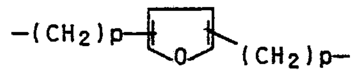 ,

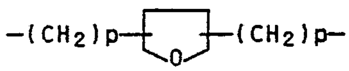 , 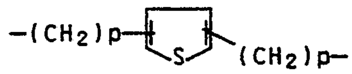 , 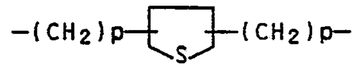 ,

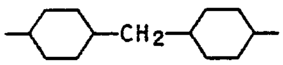 ,

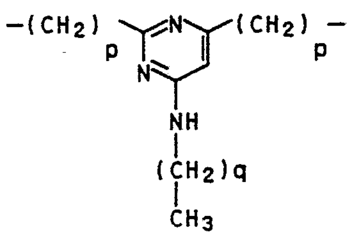 , 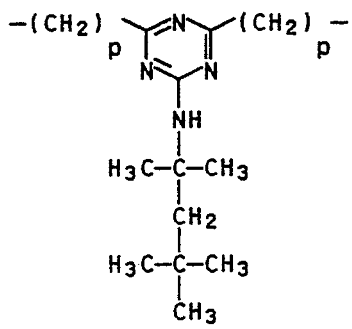 , 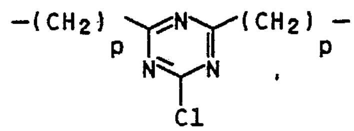 ,

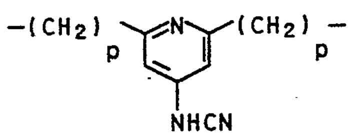 , 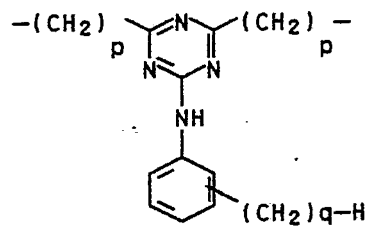 ,

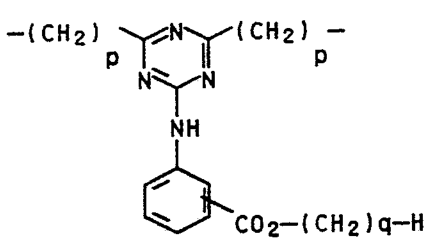 , 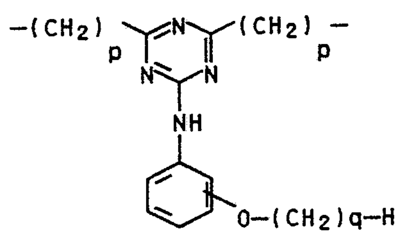 ,

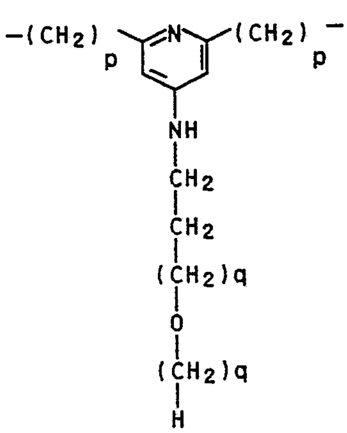

$-(-CH_2CH_2-O-)_p-$ , 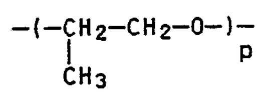 ,

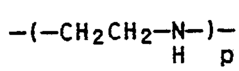 , 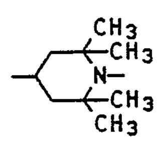 ,

wobei in den vorstehenden Formeln p für 1 bis 20 steht.

Der in Verbindung mit den Restedefinitionen gebrauchte Begriff "gegebenenfalls substituiert" umfaßt die vorstehend erläuterten Bedeutungsmöglichkeiten. Er umfaßt insbesondere Substituenten, wie $C_1$-$C_6$-Alkyl, $C_1$-$C_6$-Alkoxy, $C_2$-$C_{12}$-Carbonester, $C_1$-$C_{12}$-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy, (Poly)amino.

Der Begriff "C-Acyl" steht beispielsweise für $C_1$-$C_{20}$-Acyl, insbesondere für Reste der Formel

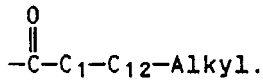

Die Herstellung der erfindungsgemäßen Verbindungen ergibt sich aus der FR-A-2 291 203.

So kann man die Verbindungen der allgemeinen Formel (I) durch Umsetzung von primären Aminen $R^3$-$NH_2$, Aminocarbonsäureestern, Aminocarbonsäureamiden, Aminosulfonsäuren, Aminosulfonsäureestern oder Aminosulfonsäureamiden, oder entsprechenden Verbindungen, mit Verbindungen der allgemeinen Formel (II) herstellen.

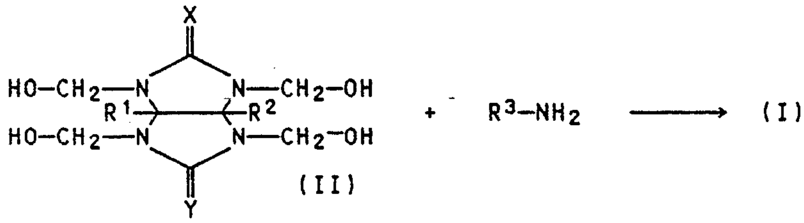

Die Verbindungen der allgemeinen Formel (II) können dabei auch in situ durch Reaktion von Verbindungen der allgemeinen Formel (III) mit Formaldehyd oder einer Formaldehydquelle hergestellt werden.

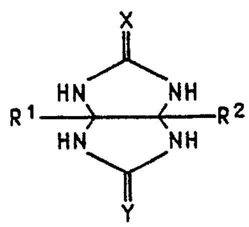

(III).

Die ergfindungsgemäßen Verbindungen besitzen außerordentlich gute stabilisierende Eigenschaften, haben keine Eigenfarbe, sind gut verträglich mit organischen Polymeren und zeigen einen niedrigen Dampfdruck. Sie sind darüber hinaus auch wertvolle Zwischenprodukte für die Herstellung von Stabilisatoren, Pflanzenschutzmitteln, Pharmaka und Polymeren.

Verbindungen der allgemeinen Formel (I), worin B-für -$CO_2R^6$ ($R^6$ = $C_1$-$C_{22}$-Alkyl) steht, können beispielsweise durch Aminolyse in Verbindungen mit

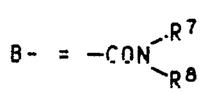

überführt werden.

Die erfindungsgemäßen Verbindungen können in Form der freien Basen oder als Salze vorliegen. Geeignete Anionen stammen z. B. von anorganischen Säuren und insbesondere organischen Carbonsäuren sowie organischen Sulfonsäuren.

Anorganische Anionen sind z.B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanonat, Cyclohexanonat,

Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen eignen sich zum Stabilisieren von organischem Material, speziell von Kunststoffen, gegen den Abbau durch Licht und Wärme. Sie werden den zu stabilisierenden Kunststoffen in einer Konzentration von 0,01 bis 5 Gew.%, vorzugsweise 0,02 bis 1 Gew.% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-$\beta$-(3,5-di-tert.-butly-4-hydro-xyphenyl)-propionat, 1,1,3-Tris-(2-methyl-4-hydroxy-5-tert.-butyl-phenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[$\beta$-(3,5-di-tert.-butyl-4-hydroxyphenyl)-propionyloxy-ethyl]-isocyanurat, 1,3,5-Tris-(2,6,di-methyl-3-hydroxy-4-tert.-butylbenzyl)-isocyanurat, Pentaerythrit-tetrakis-[$\beta$-(3,5-di-tert.-butyl-4-hydroxy-phenyl)-propionat], erwähnt.

Als phosphorhaltige Antioxidantien seien beispielsweise Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit, erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-($\beta$-laurylthiopropionat), Pentaerythrittetrakis-($\beta$-hexylthiopropionat), erwähnt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, $\alpha$-Cyanozimtsäurederivate, Nickelverbindungen oder Oxalsäuredianilide.

Als organische Polymere, die durch die erfindungsgemäßen Verbindungen stabilisiert werden können, seine beispielsweise genannt:

Polymere von Mono-und Diolefinen, wie z.B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono-oder Diolefinen oder Mischungen der genannten Polymeren;

Copolymerate von Mono-oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder $\alpha$-Methylstyrol mit Dienen oder Acrylderivaten, wie z.B. Styrol-Butadien, Styrol-Acrylnitril, Styrol-Ethylmethacrylat, Styrol-Butadien-Ethylacrylat, Styrol-Acrylnitril-Methacrylat;

ABS-, MBS-oder ähnliche Polymere;

Halogenhaltige Polymere, wie z.B. Polyvinylchlorid, Polyvinylfluorid, Polyvinylidenfluorid sowie dere Copolymere;

Polymere die sich von $\alpha,\beta$-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können in fester oder gelöster Form dem Lack zugesetzt

werden. Ihre gute Löslichkeit in Lacksystemen ist dabei von besonderem Vorteil.

Bevorzugt ist die Verwendung der erfindungsgemäßen Verbindungen in Polyolefinen, vorzugsweise Ethylen-und Propylenpolymerisaten.

Die Erfindung wird durch die nachstehenden Beispiele weiter erläutert.

Beispiel 1 –

In 70 ml Wasser löst man 26.2 g 6-Aminohexansäure und 52 g einer 50 %igen wäßrigen Tetramethylacetylendiharnstofflösung, gibt 16 g 50 %ige Natronlauge zu und erhitzt 2 h zum Rückfluß. Nach dem Abkühlen stellt man mit konzentrierter Salzsäure einen pH-Wert von 3 bis 5 ein. Man saugt den ausgefallenen Niederschlag ab und kristallisiert aus Wasser um. Man isoliert die Verbindung der Formel

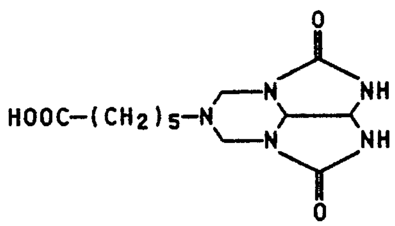

als farblose Kristalle vom Schmp. 262°C (Zers.)

ber. C 48,5 H 6,4 N 23,6 O 21,5
gef. C 48,4 H 6,6 N 23,5 O 21,7

Beispiel 2

86 g Diethylaminopropylamin und 173 g einer 50 %igen wäßrigen Lösung von Tetramethylacetylendiharnstoff werden in 260 ml Wasser 2 h zum Rückfluß erhitzt. Nach dem Abdestillieren des Lösungsmittels wird aus iso-Propanol umkristallisiert. Man isoliert die Verbindung der Formel

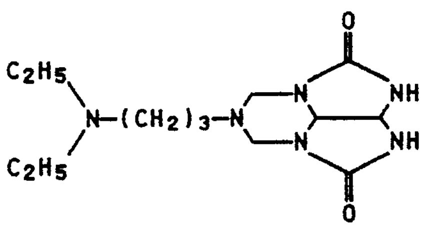

als farblose Kristalle vom Schmp. 270°C (Zers.)

ber. C 52,7 H 8,1 N 28,3 O 10,8
gef. C 52,7 H 8,2 N 27,8 O 11,0

## Ansprüche

1. Verbindungen der allgemeinen Formel (I)

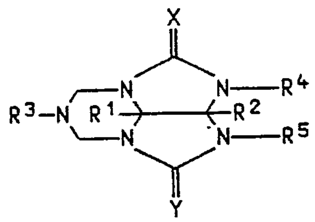

(I),

worin

$R^1$ und $R^2$ unabhängig voneinander Wasserstoff, $C_1$-$C_8$-Alkyl, $C_5$-$C_8$-Cycloalkyl, $C_7$-$C_{12}$-Aralkyl, $C_7$-$C_{14}$-Cycloalkylalkyl, Aryl oder Carbonester bedeuten oder $R^1$ und $R^2$ zusammen eine Tetra-, Penta-oder Hexamethylengruppierung oder einen gegebenenfalls substituierten Rest der Formel

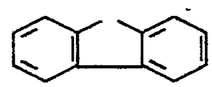

darstellen,

X und Y unabhängig voneinander für Sauerstoff, Schwefel oder $NR^{10}$ stehen, wobei $R^{10}$ Wasserstoff, $C_1$-$C_8$-Alkyl oder $C_7$-$C_{12}$-Aralkyl ist, und

$R^3$ für Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, $C_3$-$C_{12}$-Alkinyl, Hydroxy, einen nichtaromatischen, gegebenenfalls substituierten Heterocyclus, einen $C_1$-$C_{22}$-Alkylrest, der durch einen Heterocyclus substituiert ist, einen $C_1$-$C_{22}$-Alkylrest, der durch Amino, Hydroxy, Chlor, Brom, Jod, Sulfon, Sulfoxid, eine Urethangruppe, eine Harnstoffgruppe, Carboxyl, Carbonester und/oder gegebenenfalls substituiertes Carbamoyl substituiert ist, oder für die Gruppierung B-A-steht, worin -A-ein Brückenglied und B-einen Thiol-oder Cyanrest oder eine Gruppe der Formeln

$-CO_2R^6$ ; 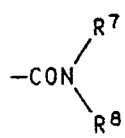 ; $-SO_3R^7$ ; 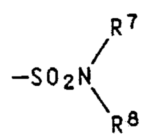 ;

bedeuten, worin

$R^6$ für Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl steht,

$R^7$ und $R^8$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^7$ und $R^8$ gemeinsam mit dem Atom, an das sie gebunden sind, ein 3 bis 12-gliedriges Ringsystem bilden, und

$R^4$ und $R^5$ unabhängig voneinander für Wasserstoff oder $CH_2OR^9$ stehen, worin $R^9$ die Bedeutungen Wasserstoff oder gegebenenfalls substituiertes $C_1$-$C_{22}$-Alkyl, das durch Ethersauerstoff, Schwefel oder Stickstoff unterbrochen sein kann, besitzt,

sowie die Säureadditionssalze und Hydrate dieser Verbindungen.

2. Verbindungen nach Anspruch 1, worin $R^1$ und/oder $R^2$ für Wasserstoff, Methyl oder Phenyl stehen.

3. Verbindungen nach einem der Ansprüche 1 oder 2, worin X und Y für Sauerstoff stehen.

4. Verwendung der Verbindungen nach einem der Ansprüche 1 bis 3 zum Stabilisieren von organischem Material, insbesondere von Kunststoffen und Lacken.

5. Verwendung nach Anspruch 4 zum Stabilisieren von Polyolefinen und Polyamiden.

6. Verwendung nach einem der Ansprüche 4 oder 5 zum Licht-und Wärmeschutz oder als Metallionendesaktivator.

7. Verwendung der Verbindungen nach einem der Ansprüche 1 bis 3 als Zwischenprodukt zur Herstellung anderer Stabilisatoren.

8. Stabilisiertes organisches Material, enthaltend eine Verbindung nach einem der Ansprüche 1 bis 3.